(19) Europäisches Patentamt
European Patent Office
Office européen des brevets

(11) Veröffentlichungsnummer: **0 081 157**
**B1**

(12) **EUROPÄISCHE PATENTSCHRIFT**

(45) Veröffentlichungstag der Patentschrift: **02.01.85**

(51) Int. Cl.³: **C 07 C 103/76,** C 07 C 102/04

(21) Anmeldenummer: **82110924.6**

(22) Anmeldetag: **26.11.82**

(54) **Verfahren zur Herstellung von Nitroarylcarbonsäure-nitroarylamiden.**

(30) Priorität: **09.12.81 DE 3148696**

(43) Veröffentlichungstag der Anmeldung:
**15.06.83 Patentblatt 83/24**

(45) Bekanntmachung des Hinweises auf die Patenterteilung:
**02.01.85 Patentblatt 85/1**

(84) Benannte Vertragsstaaten:
**CH DE FR GB IT LI**

(56) Entgegenhaltungen:
**FR - A - 2 113 418**
**FR - A - 2 192 819**

**CHEMICAL ABSTRACTS, Band 91, Nr. 1, 2. Juli 1979, Seite 469, Nr. 5008x, Columbus, Ohio, USA E.L. VULAKH et al.: "Use of phosphorus oxychloride for the synthesis of aromatic carboxylic acid chlorides"**

(73) Patentinhaber: **BAYER AG, Konzernverwaltung RP Patentabteilung, D-5090 Leverkusen 1 Bayerwerk (DE)**

(72) Erfinder: **Müller, Nikolaus, Dr., Walter-Flex-Strasse 32, D-5090 Leverkusen (DE)**
Erfinder: **Blank, Heinz Ulrich, Dr., Am Geusfelde 35, D-5068 Odenthal (DE)**

**EP 0 081 157 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft ein Verfahren zur Herstellung von Nitroarylcarbonsäure-nitroarylamiden aus Nitroarylcarbonsäuren und Nitroarylaminen.

Es ist bereits bekannt, Nitroarylcarbonsäure-nitroarylamide durch Reaktion von Nitroaroylchloriden mit Nitroarylaminen herzustellen (US 3926922; BE 672361; Bull. Soc. Chim. France 1960, 1956). Diese Verfahren sind zweistufig und erfordern primär die getrennte Herstellung des Säurechlorids aus der zugrundeliegenden Nitroarylcarbonsäure und einem Chlorierungsmittel.

Die direkte Kondensation der zugrundeliegenden Arylcarbonsäure mit dem Arylamin ist weiterhin für den Fall bekannt, daß der die Carboxylgruppe tragende Arylkern zusätzlich mit einer Hydroxygruppe, insbesondere mit einer ortho-ständigen Hydroxygruppe, substituiert ist. So ist aus US 3 221 052 die Herstellung von Salicylanilid aus Salicylsäure und Anilin in inerten Kohlenwasserstoff- oder Chlorkohlenwasserstoff-Lösungsmitteln, im Temperaturbereich von 75 bis 180°C in Gegenwart von $PCl_3$ bekannt. Nach Abkühlung des Reaktionsgemisches wird dieses mit einer Lösung von Alkalimetallhydroxiden oder -carbonaten versetzt. Die abgetrennte organische Schicht, die das gewünschte Salicylanilid enthält, wird sodann, beispielsweise durch Wasserdampfdestillation, in das inerte organische Lösungsmittel und das gewünschte Salicylanilid getrennt. Die Reinheit eines so hergestellten aromatischen Hydroxycarbonsäure-arylamids ist für viele Weiterverarbeitungen, beispielsweise zur Herstellung von Azopigmenten, vielfach nicht ausreichend. Zur Verbesserung des Reinheitsgrades wird daher beispielsweise in DE-AS 2 405 986 vorgeschlagen, das Reaktionsgemisch aus der Herstellung solcher aromatischen Hydroxycarbonsäure-arylamide oder das Reaktionsprodukt selbst mit einer Aminopolycarbonsäure als Chelatbildner zu behandeln. Für den Fall, daß diese Behandlung auf das Reaktionsgemisch angewandt wird, wird das inerte organische Lösungsmittel ebenfalls mit Wasserdampf aus dem Reaktionsgemisch abdestilliert.

Es ist ferner bekannt, bei der Herstellung von 2-Hydroxy-3-naphthoesäure-anilid die Kristallgröße dadurch zu erhöhen und damit die Filtration zu verbessern, daß der Reaktionsmischung ein Dialkylarylamin zugesetzt wird (Japanische Patentmeldung 55-120547).

Die Kondensation einer Nitroarylcarbonsäure mit einem Nitroarylamin in einem inerten Lösungsmittel mit Phosphortrichlorid wird in der japanischen Patentanmeldung 72-35 900 für den Fall als erfolgreich beschrieben, daß dem Reaktionsgemisch Säureamide, beispielsweise Dimethylformamid zugesetzt werden. Setzt man anstelle von Dimethylformamid Triethylamin zum Reaktionsgemisch, so sinkt die Ausbeute drastisch auf unter 50%. Ohne jeden Zusatz werden Nitroarylcarbonsäure-nitroarylamide nur in Ausbeuten von etwa 10 bis 20% erhalten, wobei die Reaktion in einigen Fällen, wie dem 4,4'-Dinitrobenzanilid, zu teerigen Massen führt.

Die Varianten des zuletzt genannten Verfahrens werden wegen der schlechten Löslichkeit von Nitroarylverbindungen in Suspension durchgeführt. Hierbei sind, um eine bessere Rührbarkeit des Gemisches zu erreichen, hohe Verdünnungen und verlängerte Reaktionszeiten erforderlich, woraus eine schlechte Raum/Zeit-Ausbeute resultiert. Weiterhin kann beim Arbeiten in Suspensionen die Reinheit der Endprodukte durch Einschlüsse an Ausgangskomponenten beeinflußt werden. Beim Arbeiten mit der Kombination Dimethylformamid/Phosphortrichlorid ist schließlich mit der Bildung des stark karcinogenen Dimethylcarbamoylchlorids (DIMCA) als Nebenprodukt zu rechnen.

Es wurde nun ein Verfahren zur Herstellung von Nitroarylcarbonsäure-Nitroarylamiden der Formel

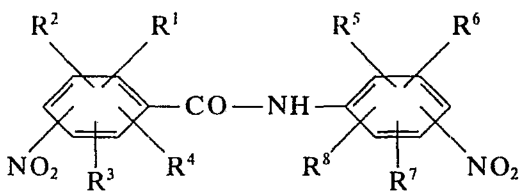

(I)

in der

$R^1$ bis $R^8$ unabhängig voneinander Wasserstoff, Alkyl, Halogen, Alkoxy oder Alkylthio sein können und $R^1$ und $R^5$ zusätzlich und ebenfalls unabhängig voneinander Nitro bedeuten können,

in einem inerten Lösungsmittel bei erhöhter Temperatur gefunden, das dadurch gekennzeichnet ist, daß man Nitroarylcarbonsäuren der Formel

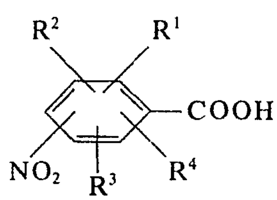

(II)

in der

$R^1$ bis $R^4$ die angegebene Bedeutung haben,

mit Nitroarylaminen der Formel

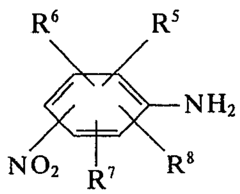

(III)

in der

$R^5$ bis $R^8$ die angegebene Bedeutung haben,

bei 50 bis 200°C in Gegenwart von 0,33 bis 2 Mol Phosphoroxichlorid und bei einem Anteil von 1 bis 100 Gew.-% an tertiären Aminen der Formel

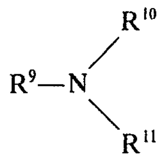

(IV)

in der

$R^9$ Alkyl, Cycloalkyl, gegebenenfalls substituiertes Aryl oder gegebenenfalls substituiertes Aralkyl und

$R^{10}$ und $R^{11}$ unabhängig voneinander niedriges Alkyl oder gegebenenfalls substituiertes Aryl bedeuten, wobei weiterhin $R^{10}$ und $R^{11}$ gemeinsam eine gegebenenfalls durch Heteroatome unterbrochene Alkylenkette darstellen können,

am Gesamtgewicht der Lösungsmittel umsetzt.

Als Alkyl sei beispielsweise ein geradkettiger oder verzweigter aliphatischer Rest mit 1 bis 12, bevorzugt 1 bis 8, besonders bevorzugt 1 bis 4 C-Atomen genannt, wie Methyl, Etyhl, Propyl, Isopropyl, Butyl, Isobutyl, Hexyl, Isohexyl, Octyl, Decyl oder Dodecyl. Alkyl mit 1 bis 4, ganz besonders bevorzugt 1 bis 2 Kohlenstoffatomen, wird hierbei als niederes Alkyl verstanden.

Als Halogen sei beispielsweise Fluor, Chlor, Brom oder Jod, bevorzugt Fluor, Chlor oder Brom, besonders bevorzugt Chlor oder Brom, genannt.

Als Alkoxy sei beispielsweise der Rest eines alipatischen Alkohols mit 1 bis 4, bevorzugt 1 bis 2 C-Atomen genannt, wie Methoxy, Ethoxy, Propoxy, Isopropyloxy, Butoxy oder Isobutyloxy.

Als Alkylthio sei ein Schwefelanalogon der genannten Alkoxygruppen genannt, in dem also lediglich der Sauerstoff gegen Schwefel ausgestauscht ist.

Als Aryl sei beispielsweise Phenyl, Naphthyl, Diphenyl oder Anthryl, bevorzugt Phenyl, genannt.

Als Aralkyl seien beispielsweise Gruppen genannt, die eine der genannten Arylgruppen, bevorzugt die Phenylgruppe, und einen aliphatischen Teil mit 1 bis 4, bevorzugt 1 bis 2 C-Atomen enthalten, wie Benzyl, $\alpha$-Phenyl-ethyl, $\beta$-Phenyl-ethyl, Naphthylmethyl, Naphthylethyl, Anthrylmethyl, Anthrylethyl, bevorzugt Benzyl. Für den Fall, daß eine Aryl- oder Aralkylgruppe substituiert ist, kommen hierfür beispielsweise die genannten niederen Alkylgruppen, Halogen oder Alkoxy in Frage.

Als Cycloalkyl sei beispielsweise solches mit 4 bis 8, bevorzugt mit 5 bis 6 Ring-C-Atomen genannt, das gegebenenfalls mit niederem Alkyl oder Halogen substituiert sein kann, sie Cyclobutyl, Cyclopentyl, Methyl-cyclopentyl, Ethyl-cyclopentyl, Cyclohexyl, Methyl-cyclohexyl, Ethyl-cyclohexyl, Cycloheptyl, Cyclooctyl.

Für den Fall, daß $R^{10}$ und $R^{11}$ gemeinsam eine Alkylenkette bilden, sei eine solche mit 4 bis 6 Kettengliedern erwähnt, wie Tetramethylen, Pentamethylen oder Hexamethylen, die ihrerseits durch niederes Alkyl oder Halogen substituiert sein kann. Gemeinsam mit dem N-Atom, das die aus $R^{10}$ und $R^{11}$ gemeinsam gebildete Alkylenkette trägt, gelangt man so in die Reihe heterocyclischer Systeme, wie Pyrrolidin oder Piperidin. Die Alkylenkette kann weiterhin durch Heteroatome, wie Stickstoff, Sauerstoff oder Schwefel unterbrochen sein, wodurch man in analoger Weise zu heterocyclischen Systemen, wie Pyrazolidin, Imidazolidin, Oxazolidin, Thiazolidin, Piperazin, Morpholin oder Thiomorpholin, gelangt.

Als Nitroarylcarbonsäuren seien beispielsweise genannt: 2-Nitro-, 3-Nitro- und 4-Nitrobenzoesäure, 4-Chlor-2-nitrobenzoesäure, 3-Chlor-nitrobenzoesäure, 6-Chlor-2-nitrobenzoesäure, 5-Chlor-2-nitrobenzoesäure, 2-Chlor-, 4-Chlor-, 5-Chlor und 6-Chlor-3-nitrobenzoesäure, 2-Chlor- und 3-Chlor-4-nitrobenzoesäure, 2,4-Dinitro- und 3,5-Dinitrobenzoesäure, 3-Methyl-, 4-Methyl-, 5-Methyl- und 6-Methyl-3-nitrobenzoesäure, 2-Methyl- und 3-Methyl-4-nitrobenzoesäure, 6-Fluor-3-nitrobenzoesäure, 6-Brom-3-nitrobenzoesäure, 2-Methoxy-4-nitrobenzoesäure, 2-Methoxy-3-nitrobenzoesäure, 2-Methoxy-5-nitrobenzoesäure.

Als Nitroarylcarbonsäuren seien bevorzugt solche der Formel

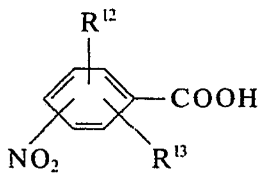

(V)

genannt, in der

$R^{12}$ und $R^{13}$ unabhängig voneinander Wasserstoff, Alkyl oder Halogen bedeuten, wobei einer der Reste $R^{12}$ und $R^{13}$ zusätzlich Nitro bedeuten kann.

Als Nitroarylcarbonsäuren seien besonders bevorzugt solche der Formel

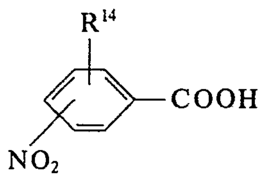

(VI)

genannt, in der

$R^{14}$ Wasserstoff, Alkyl, Halogen oder Nitro bedeutet.

Als Nitroarylamine seien beispielsweise genannt: 2-Nitranilin, 3-Nitranilin, 4-Nitranilin, 5-Chlor-2-nitranilin, 2-Chlor-4-nitranilin, 6-Chlor-3-nitranilin, 6-Nitro-m-toluidin, 4-Chlor- und 6-Chlor-2-nitranilin, 2-Chlor-, 4-Chlor- und 5-Chlor-m-nitranilin, 3-Chlor-4-nitranilin, 3-Nitro-, 4-Nitro- und 5-Nitro-o-toluidin, 2-Nitro-, 4-Nitro- und 5-Nitro-m-toluidin, 2- und 3-Nitro-p-toluidin, 2,4- und 3,5-Dinitroanilin, 2-Fluor-5-nitroanilin, 2-Brom-5-nitroanilin, die isomeren o-, m- und p-Nitroanisidine.

Als Nitroarylamine seien bevorzugt solche der Formel

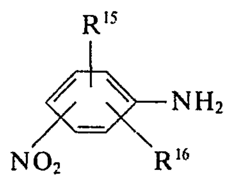

(VII)

genannt, in der

$R^{15}$ und $R^{16}$ unabhängig voneinander Wasserstoff, Alkyl oder Halogen bedeuten, wobei einer der Substituenten $R^{15}$ oder $R^{16}$ zusätzlich Nitro bedeuten kann.

Als Nitroarylamine seien besonders bevorzugt solche der Formel

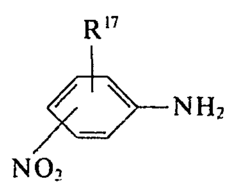

(VIII)

genannt, in der

$R^{17}$ Wasserstoff, Alkyl, Halogen oder Nitro sein kann.

Erfindungsgemäß werden 0,5 bis 5 Mol Nitroarylcarbonsäure, bevorzugt 0,7 bis 2 Mol, besonders bevorzugt etwa 1 Mol pro Mol Nitroarylamin eingesetzt.

Das erfindungsgemäße Verfahren wird in Gegenwart von 0,33 bis 2 Mol Phosphoroxichlorid, bevorzugt 0,4 bis 1,5 Mol, besonders bevorzugt 0,5 bis 1 Mol pro Mol Nitroarylcarbonsäure durchgeführt.

Die Umsetzung erfolgt bei 50 bis 200°C, bevorzugt bei 80 bis 170°C, besonders bevorzugt bei 100 bis 150°C.

Als inerte Lösungsmittel können beispielsweise aliphatische oder aromatische Kohlenwasserstoffe oder Halogenkohlenwasserstoffe eingesetzt werden. Bevorzugt wählt man solche inerte Lösungsmittel, deren Siedepunkte innerhalb des oben angegebenen Temperaturbereiches liegen. Als solche seien beispielsweise genannt: Höhersiedende Paraffinfraktionen, Isododecan, Toluol, Xylol, Ethylbenzol, Mesitylen, Chlorbenzol, die isomeren Dichlorbenzole oder die isomeren Trichlorbenzole, Brombenzol, Dibrombenzole. Inerte Lösungsmittel von der genannten Art können einzeln oder als Gemisch eingesetzt werden.

Zu den inerten Lösungsmitteln für das erfindungsgemäße Verfahren zählen weiterhin tertiäre Amine der o. g. Formel (IV). Solche tertiären Amine werden in besonderer Weise genannt, da erfindungsgemäß das einzusetzende Lösungsmittelgemisch mindestens einen kleinen Teil solcher tertiären Amine neben den o. g. Kohlenwasserstoffen oder Chlorkohlenwasserstoffen enthält, aber auch vollständig aus einem oder mehreren solcher tertiären Amine bestehen kann. Als Beispiele für solche tertiären Amine seien genannt: N,N-Dimethylanilin, N,N-Diethylanilin, N,N-Di-n-propylanilin, N,N-Dibutylanilin, 3-Methyl-N,N-dimethylanilin, 4-Methoxy-N,N-dimethylanilin, Trimethylanilin, Triethylamin, Tri-n-propylamin, Tributylamin, Dimethyl-butylamin, Diethyl-butylamin, Dimethyl-octylamin, Dimethyl-decylamin, Dimethyl-dodecylamin, Dimethyl-cyclohexylamin, Diethyl-cyclohexylamin, Dicyclohexyl-ethylamin, N-Methyl-morpholin, N-Methyl-pyrrolidin, N-Methyl-piperidin, N-Ethyl-pyrrolidin, N-Ethyl-piperidin, N-Ethyl-morpholin, N-Methyl- und N-Ethyl-diphenylamin, 2-Chlor-, 3-Chlor- und 4-Chlor-N,N-dimethylanilin.

Unter den genannten tertiären Aminen werden solche bevorzugt eingesetzt, deren Siedepunkte innerhalb des o. g. Temperaturbereiches liegen. Dadurch ist, beispielsweise bei Anwendung eines einfachen Rückflußkühlers, eine Durchführung des erfindungsgemäßen Verfahrens bei Normaldruck möglich. Darüber hinaus ist es jedoch bei Anwendung eines höheren Druckes möglich, das erfindungsgemäße Verfahren auch mit Hilfe der niedriger siedenden aliphatischen tertiären Amine durchzuführen.

Als tertiäre Amine werden bevorzugt solche der Formel

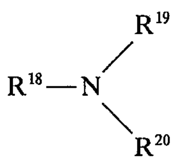

(IX)

eingesetzt, in der

$R^{18}$ für gegebenenfalls substituiertes Phenyl und
$R^{19}$ und $R^{20}$ unabhängig voneinander für Methyl oder Ethyl stehen.

Von den genannten tertiären Aminen kann sowohl eines als auch ein Gemisch von mehreren eingesetzt werden.

Erfindungsgemäß beträgt der Anteil des oder der tertiären Amine am Gesamtgewicht aller Lösungsmittel 1 bis 100 Gew.-%, bevorzugt 2 bis 100 Gew.-%, besonders bevorzugt 4 bis 100 Gew.-%. Unter dem Anteil von 4 bis 100 Gew.-% an tertiären Aminen sei ganz besonders bevorzugt ein Anteil von 4 bis 50 Gew.-% und in ganz besonders vorteilhafter Weise ein solcher von 5 bis 10 Gew.-% genannt. Bei dieser Variante finden größere Mengen der o. g. Kohlenwasserstoffe oder Chlorkohlenwasserstoffe als ergänzende Lösungsmittel Verwendung. Dies kann aus Kostengründen vorteilhaft sein.

Unter dem Anteil von 4 bis 100 Gew.-% an tertiären Aminen am Gesamtlösungsmittel sei in weiterer ganz besonders bevorzugter Weise ein Anteil von 50 bis 100% an tertiärem Amin, in ganz besonders vorteilhafter Weise ein 100%iger Anteil an tertiärem Amin oder am Gemisch mehrerer tertiärer Amine genannt. Diese Variante kann wegen des allgemeinen höheren Preises der Amine gegenüber den inerten Kohlenwasserstoffen oder Chlorkohlenwasserstoffen kostenaufwendiger sein, bringt aber dadurch einen Vorteil, daß auch bei der Anwendung kleinerer Lösungsmittelmengen und damit höherer Substratkonzentrationen bis zum Ende der Umsetzung eine homogene Lösung mit den dabei verbundenen Vorteilen der besseren Rührfähigkeit und der besseren Beherrschung der Temperatur vorliegt. Bei einem 100%igen Anteil von tertiären Aminen oder 100% nur eines tertiären Amins ergeben sich weiterhin Vorteile bei einer gegebenenfalls nötigen Reinigung oder Wiedergewinnung dieses tertiären Amins.

Die Gesamtmenge an Lösungsmittel, bestehend aus einem oder mehreren tertiären Aminen und gegebenenfalls den genannten inerten Kohlenwasserstoffen oder Chlorkohlenwasserstoffen, beträgt im allgemeinen 0,3 bis 2 l, bevorzugt 0,4 bis 1 l, besonders bevorzugt 0,5 bis 0,8 l pro Mol Nitroarylcarbonsäure.

Das erfindungsgemäße Verfahren wird im allgemeinen wie folgt durchgeführt: Die Nitroarylcarbonsäure und das Nitroarylamin werden zusammen im Lösungsmittel vorgelegt, auf 50 bis 120°C erhitzt und das Phosphoroxidchlorid bei 50 bis 120°C zugetropft. Nach vollständiger Zugabe wird bei einer Temperatur bis zu 200°C nachgerührt. Nach dem Abkühlen der Reaktionsmischung wird diese mit wäßriger Natronlauge bis zur Neutralisation versetzt, wobei beispielsweise ein pH-Wert von etwa 4 bis 11, bevorzugt 7 bis 9, erreicht wird. Hierbei fällt das gewünschte Nitroarylcarbonsäure-nitroarylamid aus, wird anschließend abfiltriert, abzentrifugiert oder abdekantiert und mit heißem Wasser und/oder verdünnter Salzsäure nachgewaschen.

Das anfallende Filtrat läßt sich sodann in eine wäßrige und eine organische Phase trennen. Die organische Phase kann in bekannter Weise, beispielsweise durch kurzes Andestillieren, vom größten Teil des Wassers befreit werden.

In besonders einfacher Weise gestaltet sich das erfindungsgemäße Verfahren für den Fall, daß als tertiäres Amin ein solches mit mindestens einer aromatischen Gruppe eingesetzt wird. Hierbei ist die Trennung in die organische und in die wäßrige Phase besonders stark ausgeprägt. Diese organische Phase, die auch im wesentlichen alles erfindungsgemäß eingesetzte tertiäre Amin enthält, kann ohne weitere Aufarbeitung wieder in das Verfahren eingesetzt werden. Vorteilhaft wird man bei dem Wiedereinsetzen eines solchen wasserfeuchten organischen, das tertiäre Amin enthaltenden Lösungsmittels bzw. beim Wiedereinsetzen des wasserfeuchten 100%igen tertiären Amins vor dem Zusatz des Phosphoroxichlorids durch kurzes Andestillieren dieses Reaktionsmedium vom Wasser weitgehend befreien. Diesen Vorteil des Einsatzes von wasserfeuchtem Lösungsmittel kann man weiterhin auch ausdehnen auf den Einsatz einer wasserfeuchten Nitroarylcarbonsäure bzw. eines wasserfeuchten Nitroarylamins. Solche wasserfeuchten Einsatzprodukte können beispielsweise als wasserfeuchter Filterkuchen von ihrer vorangegangenen Herstellung unmittelbar eingesetzt werden. In all diesen genannten Fällen wird man das Wasser durch kurzes Andestillieren weitgehend entfernen. Um den störenden Einfluß des danach verbliebenen restlichen Wassers auszuschalten, kann man vorteilhafterweise über die Mindestmenge von 0,33 Mol Phosphoroxichlorid pro Mol Nitroarylcarbonsäure hinaus einen Zuschlag an Phosphoroxichlorid einsetzen. Ein solcher geringer zusätzlicher Einsatz von Phosphoroxichlorid wird durch die Vorteile der besseren Handhabung wasserfeuchter Ausgangsstoffe und Lösungsmittel weit aufgewogen.

Das erfindungsgemäße Verfahren ermöglicht eine einstufige Durchführung der Herstellung der Nitroarylcarbonsäure-nitroarylamide und vermeidet so den Umweg über die thermisch wenig stabilen Nitroaroylchloride. Weiterhin wird der Einsatz des arbeitshygienisch bedenklichen Dimethylformamids vermieden. Die Reaktionsansätze können bei höheren Konzentrationen, ausreichender Rührbarkeit der Reaktionsmischung, bei Vermeidung von Teerbildung und verlängerten Reaktionszeiten und bei Erzielung hoher Raum/Zeit-Ausbeuten durchgeführt werden. Die Aufarbeitung und Rückgewinnung des Lösungsmittels erfolgt ohne aufwendige Wasserdampfdestillation. Die gewünschte Nitroarylcarbonsäure-nitroarylamide fallen weiterhin ohne aufwendige Reinigungsoperationen in hoher Ausbeute und hoher Reinheit an.

Es ist überraschend, daß die durch Nitrogruppen substituierten Substrate, die nach den üblichen Direktkondensationen nicht oder nur in sehr geringen Ausbeuten zu den Amiden umgesetzt werden können, in glatter Reaktion zu hohen Ausbeuten bei hoher Reinheit die Amide ergeben. Dies ist umso erstaunlicher, als bekannt ist, daß Phosphoroxichlorid als Kondensationsmittel weniger reaktiv ist als Phosphortrichlorid.

Die Nitroarylcarbonsäure-nitroarylamide finden Verwendung als Herbizide (BE 672361) und stellen wichtige Zwischenstufen bei der Produktion der korrespondierenden Aminoarylcarbonsäure-aminoarylamide durch Reduktion, beispielsweise mit katalytisch angeregtem Wasserstoff, dar, die ihrerseits wertvolle Diazokomponenten für Farbstoffe darstellen. Hier sei beispielsweise die Verwendung des 4,4'-Diamino-benzanilids zur Herstellung von Azofarbstoffen nach DE-AS 115125 genannt. Aminoarylcarbonsäure-aminoarylamide sind weiterhin Ausgangsstoffe zur Herstellung von Polyurethan-Elastomeren (US 3926922).

## Beispiel 1

Eine Suspension von 552,4 Teilen p-Nitranilin und 668,4 Teilen p-Nitrobenzoesäure in 2000 Teilen Chlorbenzol und 180 Teilen N,N-Dimethylanilin wird auf 100—110°C erhitzt und bei dieser Temperatur 306,8 Teile Phosphoroxichlorid in einer Stunde zugetropft. Dann wird die Reaktionsmischung zum Sieden erhitzt und ca. 3 Stunden unter Rühren unter Rückfluß gehalten, bis die HCl-Entwicklung beendet ist.

Die Mischung wird auf 110°C abgekühlt, mit 800 Teilen Wasser versetzt und bei einer Kolbeninnentemperatur von 50°C mit ca. 300 Teilen 50%iger Natronlauge auf pH 8 bis 9 gebracht. Das Produkt wird abfiltriert, mit heißem Wasser, verdünnter Salzsäure und wieder mit heißem Wasser gewaschen und getrocknet. Man erhält 1120 Teile 4,4'-Dinitrobenzanilid (98,5%ig, Ausbeute 96% der theoretischen Ausbeute) F. 272—274°C.

Das Filtrat aus dem obigen Ansatz wird in seine Phasen getrennt und der organischen Phase soviel eines Chlorbenzol/N,N-Dimethylanilin-Gemisches (92 : 8) zugeschlagen, daß der Verlust durch die

Aufarbeitung ausgeglichen wird (ca. 200 Teile). Nach Zugabe von 552,4 Teilen p-Nitroanilin und 668,4 Teilen p-Nitrobenzoesäure werden durch leichtes Andestillieren am Wasserabscheider Wasserreste azeotrop entfernt, bei 100—110°C 306,8 Teile $POCl_3$ innerhalb einer Stunde zugegeben und weiter, wie oben beschrieben, verfahren.
Man erhält 1127 Teile 4,4'-Dinitrobenzanilid (98,3 %ig, Ausbeute 96,4 % der theoretischen Ausbeute).

Eine weitere Wiederverwendung des Lösungsmittelgemisches führt zu 1134 Teilen Anilid (Gehalt: 97,7 %, Ausbeute 96 % der theoretischen Ausbeute).

Beispiel 2

138,1 Teile p-Nitranilin und 167,1 Teile p-Nitrobenzoesäure werden in 436 Teilen Toluol und 50,6 Teilen Triethylamin suspendiert, auf 80°C erhitzt und während 30 Minuten mit 76,7 Teilen Phosphoroxichlorid versetzt. Dann wird noch 3 Stunden unter Rückfluß erhitzt, mit 250 Teilen Wasser und ca. 75 Teilen 50 %iger Natronlauge auf pH 8—9 gestellt und das Produkt abgesaugt, mit heißem Wasser, verdünnter Salzsäure und wieder mit heißem Wasser gewaschen und getrocknet. Ausbeute 267 Teile 4,4'-Dinitrobenzanilid (99 %ig, Ausbeute 92 % der theoretischen Ausbeute).

Vergleichsbeispiel JP 72-35 900

138,1 Teile p-Nitranilin, 167,1 Teile p-Nitrobenzoesäure, 872 Teile Toluol (1 l) und 50,6 Teile Triethylamin werden auf 80°C erwärmt und in 30 Minuten mit 84 Teilen Phosphortrichlorid versetzt. Es bildete sich eine schwer rührbare, klumpige Masse. Dann wurde 3 Stunden unter Rückfluß erhitzt, auf 80°C abgekühlt und mit 250 Teilen Wasser und 75 Teilen 50 %ige Natronlauge auf pH 8—9 gestellt. Filtration der Reaktionsmischung und Behandlung des Rohproduktes mit verdünnter Salzsäure und heißem Wasser auf der Nutsche sowie anschließendes Trocknen führt zu 232 Teilen rohem 4,4'-Dinitrobenzanilid (Gehalt 66,6 %, Ausbeute 53,8 % der theoretischen Ausbeute).

Beispiel 3

167,2 Teile p-Nitrobenzoesäure, 872 Teile und 138,1 Teile p-Nitranilin werden in 600 Teilen N,N-Dimethylanilin gelöst, auf 80°C erhitzt und die Lösung innerhalb von 15 Minuten mit 76,7 Teilen Phosphoroxichlorid bei 80—100°C versetzt. Dann wird 3 Stunden bei 120°C nachgerührt, auf Raumtemperatur abgekühlt und mit ca. 300 Teilen einer 25 %igen Natronlauge vorsichtig auf pH 8—9 gestellt. Das Reaktionsprodukt wird abfiltriert, mit verdünnter Salzsäure und Wasser gewaschen und getrocknet. Ausbeute: 261 Teile 4,4'-Dinitrobenzanilid (99 %ig), entsprechend 90 % der theoretischen Ausbeute.

Beispiel 4

167,2 Teile p-Nitrobenzoesäure und 138,1 Teile 3-Nitroanilin werden in 700 Teilen Toluol und 50 Teilen N,N-Dimethylanilin suspendiert und auf 80°C erhitzt. Bei dieser Temperatur werden 76,7 Teile Phosphoroxichlorid in 15 Minuten zugetropft und anschließend 3 Stunden unter Rückfluß erhitzt. Nach dem Abkühlen auf Raumtemperatur wird mit 300 Teilen 25 %iger wäßriger Natronlauge auf pH 8—9 gestellt, filtriert und der Filterkuchen mit verdünnter Salzsäure, Wasser und schließlich Methanol gewaschen. Ausbeute: 253 Teile 4,3'-Dinitrobenzanilid (88 % der theoretischen Ausbeute). F. 226—228°C.

Beispiel 5

41,8 Teile 4-Nitrobenzoesäure und 43,1 Teile 6-Chlor-3-nitranilin werden in 150 Teilen N,N-Dimethylanilin gelöst und bei 80—100°C mit 25,3 Teilen Phosphoroxichlorid versetzt. Zweistündiges Nachrühren bei 140°C, gefolgt von der üblichen Aufarbeitung, liefert 78 Teile (97 % der theoretischen Ausbeute) 3',4-Dinitro-6'-chlorbenzanilid F. 228—231°C.

**Patentansprüche**

1) Verfahren zur Herstellung von Nitroarylcarbonsäure-Nitroarylamiden der Formel

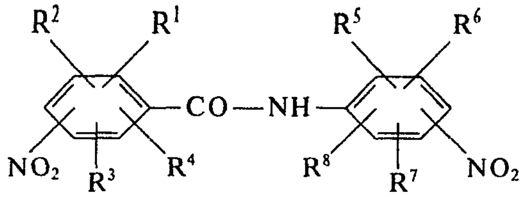

in der

$R^1$ bis $R^8$ unabhängig voneinander Wasserstoff, Alkyl, Halogen, Alkoxy oder Alkylthio sein können und $R^1$ und $R^5$ zusätzlich und ebenfalls unabhängig voneinander Nitro bedeuten können,

in einem inerten Lösungsmittel bei erhöhter Temperatur, dadurch gekennzeichnet, daß man Nitroarylcarbonsäuren der Formel

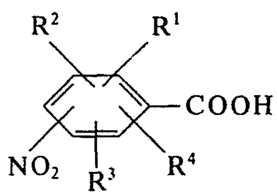

in der

$R^1$ bis $R^4$ die angegebene Bedeutung haben,

mit Nitroarylaminen der Formel

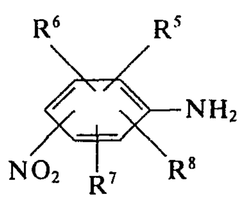

in der

$R^5$ bis $R^8$ die angegebene Bedeutung haben,

bei 50 bis 200°C in Gegenwart von 0,33 bis 2 Mol Phosphoroxichlorid und bei einem Anteil von 1 bis 100 Gew.-% an tertiären Aminen der Formel

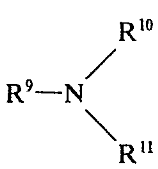

in der

$R^9$ Alkyl, Cycloalkyl, gegebenenfalls substituiertes Aryl oder gegebenenfalls substituiertes Aralkyl und

$R^{10}$ und $R^{11}$ unabhängig voneinander niedriges Alkyl oder gegebenenfalls substituiertes Aryl bedeuten, wobei weiterhin $R^{10}$ und $R^{11}$ gemeinsam eine gegebenenfalls durch Heteroatome unterbrochene Alkylenkette darstellen können,

am Gesamtgewicht der Lösungsmittel umsetzt.

2) Verfahren nach Anspruch 1, dadurch gekennzeichnet, daß eine Nitroarylsäure der Formel

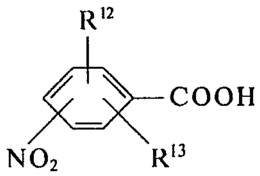

eingesetzt wird, in der

$R^{12}$ und $R^{13}$ unabhängig voneinander Wasserstoff, Alkyl oder Halogen bedeuten, wobei zusätzlich einer der Substituenten $R^{12}$ und $R^{13}$ Nitro bedeuten kann.

3) Verfahren nach Anspruch 1, dadurch gekennzeichnet, daß ein Nitroarylamin der Formel

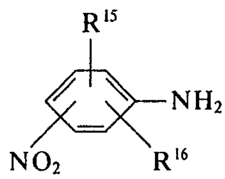

eingesetzt wird, in der

$R^{15}$ und $R^{16}$ unabhängig voneinander Wasserstoff, Alkyl oder Halogen bedeuten, wobei einer der Substituenten $R^{15}$ oder $R^{16}$ zusätzlich Nitro bedeuten kann.

4) Verfahren nach Ansprüchen 1 bis 3, dadurch gekennzeichnet, daß man 0,5 bis 5 Mol Nitroarylcarbonsäure pro Mol Nitroarylamin einsetzt.

5) Verfahren nach Ansprüchen 1 bis 4, dadurch gekennzeichnet, daß der Anteil der tertiären Amine am Gesamtgewicht des Lösungsmittels 4 bis 50 Gew.-% beträgt.

6) Verfahren nach Ansprüchen 1 bis 4, dadurch gekennzeichnet, daß der Anteil der tertiären Amine am Gesamtgewicht des Lösungsmittels 50 bis 100 Gew.-% beträgt.

7) Verfahren nach Ansprüchen 1 bis 6, dadurch gekennzeichnet, daß ein tertiäres Amin der Formel

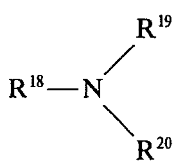

in der

$R^{18}$ gegebenenfalls substituiertes Phenyl und
$R^{19}$ und $R^{20}$ unabhängig voneinander Methyl oder Ethyl bedeuten,

eingesetzt wird.

8) Verfahren nach Ansprüchen 1 bis 7, dadurch gekennzeichnet, daß für den Fall, daß das tertiäre Amin mindestens eine Arylgruppe trägt, das Lösungsmittel bzw. das Lösungsmittelgemisch nach der wäßrigen Aufarbeitung des Reaktionsgemisches und Abfiltrieren von Feststoffen von der wäßrigen Phase abgetrennt und wieder in das Verfahren eingesetzt wird.

9) Verfahren nach Ansprüchen 1 bis 8, dadurch gekennzeichnet, daß man die Nitroarylcarbonsäure und/oder das Nitroarylamin und/oder das Lösungsmittel bzw. Lösungsmittelgemisch in wasserfeuchter Form eingesetzt, vor dem Zusatz von Phosphoroxichlorid das Wasser weitgehend abdestilliert und bei der Menge des Phosphoroxichlorids einen dem Rest-Wasser-Gehalt entsprechenden Zuschlag berücksichtigt.

## Claims

1. Process for the preparation of nitroarylcarboxylic acid nitroarylamides of the formula

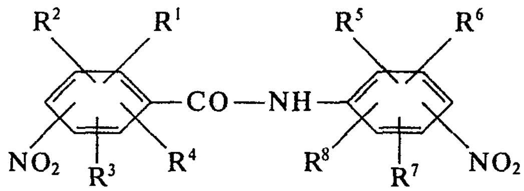

in which

$R^1$ to $R^8$ independently of one another can be hydrogen, alkyl, halogen, alkoxy or alkylthio and
$R^1$ and $R^5$ additionally and likewise independently of each other, can denote nitro,

in an inert solvent at an elevated temperature, characterised in that nitroarylcarboxalic acids of the formula

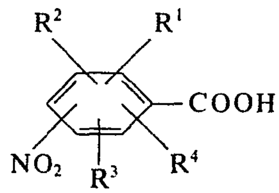

in which

$R^1$ to $R^4$ have the indicated meaning,

are reacted with nitroarylamines of the formula

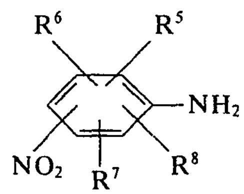

in which

$R^5$ to $R^8$ have the indicated meaning,

at 50 to 200°C in the presence of 0.33 to 2 mols of phosphorus oxychloride and of a proportion of tertiary amines of the formula

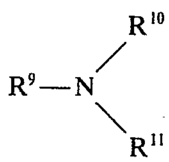

in which

$R^9$ denotes alkyl, cycloalkyl, optionally substituted aryl or optionally substituted aralkyl and
$R^{10}$ und $R^{11}$ independently of each other denote lower alkyl or optionally substituted aryl an $R^{10}$ and $R^{11}$ together can also represent and alkylene chain optionally interrupted by hetero atoms,

which constitutes 1 to 100% by weight of the total weight of the solvents.

2. Process according to Claim 1, characterised in that a nitroaryl acid of the formula

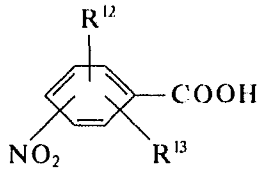

is used in which

$R^{12}$ and $R^{13}$ independently of each other denote hydrogen, alkyl or halogen, and one of the substituents $R^{12}$ and $R^{13}$ can additionally denote nitro.

3. Process according to Claim 1, characterised in that a nitroarylamine of the formula

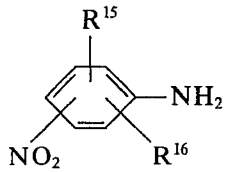

is used in which

$R^{15}$ and $R^{16}$ independently of each other denote hydrogen, alkyl or halogen and one of the substituents $R^{15}$ and $R^{16}$ can additionally denote nitro.

4. Process according to Claims 1 to 3, characterised in that 0.5 to 5 mols of nitroarylcarboxylic acid are used per mol of nitroarylamine.

5. Process according to Claims 1 to 4, characterised in that the tertiary amines constitute a proportion of 4 to 50% by weight of the total weight of the solvent.

6. Process according to Claims 1 to 4, characterised in that the tertiary amines constitute a proportion of 50 to 100% by weight of the total weight of the solvent.

7. Process according to Claims 1 to 6, characterised in that a tertiary amine of the formula

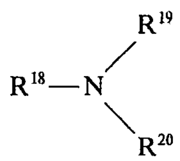

in which

$R^{18}$ denotes optionally substituted phenyl and
$R^{19}$ and $R^{20}$ independently of each other denote methyl or ethyl, is used.

8. Process according to Claims 1 to 7, characterised in that, in the case where the tertiary amine carries at least one aryl group the solvent or solvent mixture, after the aqueous working-up of the reaction mixture and after the solids have been filtered off, is separated from the aqueous phase an reused in the process.

9. Process according to Claims 1 to 8, characterised in that the nitroarylcarboxylic acid and/or the nitroarylamine and/or the solvent or solvent mixture are used in a water-moist form, the water is distilled off to a large extent before the phosphorus oxychloride is added and the amount of phosphorus oxychloride includes an additional amount corresponding to the content of residual water.

**Revendications**

1. Procédé de production de nitroarylamides d'acides nitroarylcarboxyliques, de formule

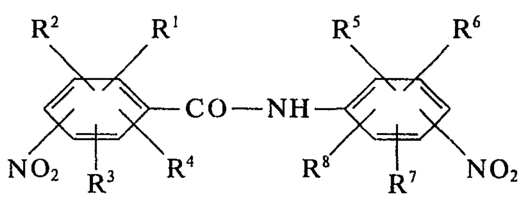

dans laquelle

$R^1$ à $R^8$ peuvent représenter, indépendamment les uns des autres, l'hydrogène, un reste alkyle, un halogène, un reste alkoxy ou alkylthio et $R^1$ et $R^5$ peuvent représenter en outre et indépendamment l'un de l'autre également, un groupe nitro,

dans un solvant inerte à température élevée, caractérisé en ce qu'on fait réagir des acides nitroarylcarboxyliques de formule

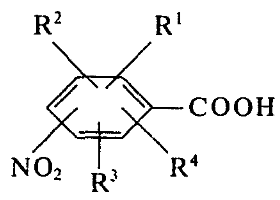

dans laquelle

$R^1$ à $R^4$ ont la définition indiquée, avec des nitroarylamines de formule

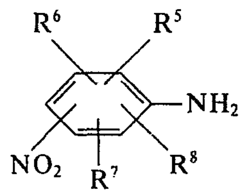

dans laquelle

$R^5$ à $R^8$ ont la définition indiquée,

à une température de 50 à 200°C en présence de 0,33 à 2 moles d'oxychlorure de phosphore à côté d'une proportion de 1 à 100% en poids d'amines tertiaires de formule

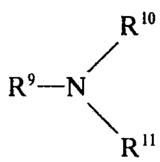

dans laquelle

$R^9$ est un groupe alkyle, cycloalkyle, aryle éventuellement substitué ou aralkyle éventuellement substitué et

$R^{10}$ et $R^{11}$ représentent , indépendamment l'un de l'autre, un groupe alkyle inférieur ou un groupe aryle éventuellement substitué, $R^{10}$ et $R^{11}$ pouvant en outre former ensemble une chaîne alkylénique interrompue par des hétéro-atomes,

par rapport au poids total des solvants.

2. Procédé suivant la revendication 1, caractérisé en ce qu'on utilise un acide nitroarylcarboxylique de formule

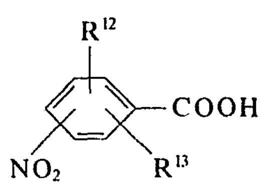

dans laquelle

$R^{12}$ et $R^{13}$ représentent, indépendamment l'un de l'autre, l'hydrogène, un groupe alkyle ou un halogène, l'un des substituants $R^{12}$ et $R^{13}$ pouvant représenter en outre un groupe nitro.

3. Procédé suivant la revendication 1, caractérisé en ce qu'on utilise une nitroarylamine de formule

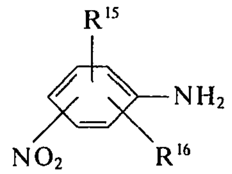

dans laquelle

$R^{15}$ et $R^{16}$ représent, indépendamment l'un de l'autre, l'hydrogène, un groupe alkyle ou un halogène, l'un des substituants $R^{15}$ et $R^{16}$ pouvant représenter en outre un groupe nitro.

4. Procédé suivant les revendications 1 à 3, caractérisé en ce qu'on utilise 0,5 à 5 moles d'acide nitroarylcarboxylique par mole de nitroarylamine.

5. Procédé suivant les revendications 1 à 4, caractérisé en ce que la proportion des amines tertiaires par rapport au poids total du solvant s'élève à 4—50% en poids.

6. Procédé suivant les revendications 1 à 4, caractérisé en ce que la proportion des amines tertiaires par rapport au poids total du solvant est de 50 à 100% en poids.

7. Prodédé suivant les revendications 1 à 6, caractérisé en ce qu'on utilise une amine tertiaire de formule

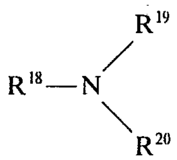

dans laquelle

$R^{18}$ désigne un groupe phényle éventuellement substitué et
$R^{19}$ et $R^{20}$ désignent, indépendamment l'un de l'autre, un groupe méthyle ou éthyle.

8. Procédé suivant les revendications 1 à 7, caractérisé en ce que, au cas où l'amine tertiaire porte au moins un groupe aryle, le solvant ou le mélange de solvants est séparé de la phase aqueuse après le traitement aquex du mélange réactionnel et la filtration des matières solides et est réutilisé dans le procédé.

9. Procédé suivant les revendications 1 à 8, caractérisé en ce qu'on utilise l'acide nitroarylcarboxylique et/ou la nitroarylamine et/ou le solvant ou le mélange de solvants sous une forme humidifiée par l'eau, on chasse largement l'eau par distillation avant l'addition de l'oxychlorure de phosphore et on prend en compte une addition correspondant à la teneur résiduelle en eau dans la quantité d'oxychlorure de phosphore.